# EUROPEAN PATENT SPECIFICATION

(11) **EP 0 196 151 B1**
(45) Date of publication and mention of the grant of the patent: **27.07.1994**
(21) Application number: 86300537.7
(22) Date of filing: 27.01.1986
(51) Int. Cl.: A61L 2/18, A01N 43/50, A01N 41/06, A01N 37/32

(54) **Disinfection of contact lenses**
Desinfektion von Kontaktlinsen
Désinfection de lentilles de contact

(30) Priority: 25.01.1985 GB 8501955
(43) Date of publication of application: 01.10.1986
(73) Proprietor: Sauflon Pharmaceuticals Limited, London SW5 9RX (GB)
(72) Inventor: Hopkinson, Andrew, Berkshire (GB); Cannell, John Seymour, Chandlers Ford, Hampshire (GB)
(74) Representative: Smart, Peter John

(56) References cited:
- FR-A- 2 256 767
- GB-A- 2 055 579
- GB-A- 2 072 371
- US-A- 3 876 768

## Description

This invention relates to compositions for use in disinfecting a contact lens and to a method of disinfecting a contact lens.

Many substances are used to disinfect contact lenses among which may be mentioned benzalkonium chloride, chlorbutol, methylparaben, chlorhexidine, propylparaben, phenol, phenylethyl alcohol, phenylmercuric nitrate and thiomersalate. In view of the conflicting requirements of pharmaceutical acceptability on the one hand and adequate lens-disinfecting ability on the other, most of these substances possess disadvantages and there are still requirements for effective contact lens disinfecting agents which will be acceptable to the eye of a wearer of a contact lens.

Our British Patent GB-A-2055579 describes and claims a lens-sterilising solution containing from 0.5 to 3 ppm by weight of dichloroisocyanurate measured as available chlorine.

US-A-3876768 discloses the disinfection of soft and hard contact lenses by means of aqueous solutions of a range of chlorine releasing materials. Similarly FR-A-2256767 disclosed disinfecting soft contact lenses by means of solutions of chlorine releasing agents including N₁N-dichlorotaurine, N₁N-dichloro-betaalanine, N₁N-dichloroisocyanuric acid, N-chlorourea, N₁N'-1,3, dichloro-5,5-dimethylhydantoin, N-chlorosuccinamide, N₁N'-dichloroglycocyamine, and N₁N'-dichloroglycine.

FR-A-2072371 discloses the use of N₁N'-dichloro-5,5 dimethylhydantoin for decontaminating contact lenses.

There remains a need for improved contact lens disinfecting materials.

Accordingly the present invention provides a method of disinfecting a contact lens which comprises treating the lens with an aqueous solution containing as a chlorine release agent 4-carboxylphenyl N-chlorosulphonamide (halazone) or a salt thereof, said chlorine release agent being present in an amount of from 0.5 to 100 ppm by weight measured as available chlorine.

According to a second aspect of the invention there is provided an isotonic buffered saline contact lens-disinfecting solution containing from 0.5 to 100 ppm by weight measured as available chlorine of halazone or a salt thereof.

According to a third aspect of the present invention there is provided a tablet or capsule for dissolution in 10 cm³ of a pharmaceutically acceptable aqueous liquid to produce a contact lens-disinfecting solution which tablet or capsule contains an amount of halazone or a salt thereof to produce a concentration of halazone of from 0.5 to 10 ppm by weight measured as available chlorine, when the tablet is dissolved in the said aqueous liquid.

According to a fourth aspect of the present invention there is provided a two-part pack which comprises a plurality of sachets each containing a predetermined quantity of a sterile, buffered saline, and a plurality of tablets or capsules, each of said tablets or capsules being soluble in the quantity of liquid contained within a said sachet and containing halazone or a salt thereof in an amount to produce with said quantity of liquid a contact lens-disinfecting solution containing from 0.5 to 100 ppm by weight of halazone measured as available chlorine.

According to a fifth aspect of the present invention there is provided a method of producing a pharmaceutically acceptable contact lens-disinfecting solution which comprises dissolving in a predetermined quantity of a sterile isotonic buffered saline a tablet or capsule containing halazone or a salt thereof in an amount to produce with said quantity of liquid a contact lens treating solution having from 0.5 to 100 ppm by weight of halazone measured as available chlorine.

More than one chlorine release agent may be employed in the present invention but the total amount present should not exceed 100 ppm by weight measured as available chlorine.

Preferably the amount of available chlorine is from 3 to 20 ppm by weight.

The solution, tablet or capsule may contain other ingredients such as are generally required to achieve satisfactory contact lens-disinfecting solutions such as chelating agents, wetting agents, catalysts, sodium chloride to assist tonicity, and buffering agents. In the case of tablets or capsules there may also be included effervescing agents to aid dissolution thereof in the aqueous carrier liquid.

Preferably the sachets containing the buffered saline are made of a synthetic plastics material. The aqueous liquid may be inserted into the sachets in a sterile condition or may be subsequently sterilised therein, for example, by heat treatment or gamma-ray irradiation; the preferred material for the sachets is polyethylene since it is practically inert to gamma-ray irradiation and is substantially free from components which may be leached into the liquid contained within the sachet. Preferred liquid dosages to be contained within a single sachet are 10 ml, 20 ml or 18 ml. It is envisaged that the sachets may be sold singly or in strips or sheets from which a single sachet may be torn off for use as required.

The tablets or capsules are preferably sold individually separated in blister packs but may be loose or in sealed containers.

As a non-limitative example of the present invention, tablets were prepared each including the following ingredients:
**4-carboxy phenyl**
**N-chlorosulphonamide (Halazone) : 0.1 mg/tablet adipic acid ) (effervescent agent) : 49.9 mg/tablet sodium bicarbonate)**
A blister or bubble pack containing 25 isolated tablets was prepared.

A polyethylene sachet or envelope was filled with 10 mls of sterile buffered saline solution of pharmaceutically acceptable strength and was then heat-sealed. Subsequently the sachet was irradiated with 2.5 Mrads of gamma-ray radiation to ensure the sterility of its contents.

One tablet was extracted from the blister pack, the sachet was opened and its contents poured into a clean container for containing a contact lens for treatment and the tablet was dissolved in the buffered saline to produce a lens-disinfecting solution having a concentration therein of 4-carboxyl phenyl N-chlorosulphonamide of 5 ppm by weight measured as available chlorine.

When contact lenses of various sorts (including hard lenses made of polymethyl methacrylate and soft lenses made of hydroxyethylmethacrylate polymers or copolymers e.g. with polyvinyl pyrrolidone or made of silicones) were immersed in this solution, they were found to be satisfactorily disinfected after a period of 2 hours and it was found that after rinsing with sterile saline the so-disinfected lenses could be inserted into the eyes of a wearer without causing discomfort or irritation.

| EXAMPLES 4-10 | | | | | | | |
|---|---|---|---|---|---|---|---|
| Example No. | 4 | 5 | 6 | 7 | 8 | 9 | 10 |
| Halazone | 0.1 | 0.2 | 0.1 | 0.2 | 0.1 | 0.2 | 0.2 |
| Adipic acid | 31.4 | 31.35 | - | - | - | - | - |
| Maleic acid | - | - | 28.95 | 28.9 | - | - | - |
| Malic acid | - | - | - | - | 27.7 | 27.7 | - |
| Sodium hydrogen phosphate | - | - | - | - | - | - | 31 |
| Sodium bicarbonate | 18.5 | 18.45 | 20.95 | 20.9 | - | - | 18.8 |
| Sodium carbonate | - | - | - | - | 22.2 | 22.1 | - |

In Examples 4-10 listed in the above table all quantities are in mgs. In each case formulations were produced as tablets containing the ingredients specified in the specified amounts. When dissolved in 10 mls isotonic buffered saline solution, the tablets so produced were found in each case to have satisfactory disinfecting properties for treating contact lenses.

## Claims

1. A method of disinfecting a contact lens which comprises treating the lens with an aqueous solution containing as a chlorine release agent 4-carboxyl phenyl N-chlorosulphonamide or a salt thereof, said chlorine release agent being present in an amount giving from 0.5 to 100 ppm by weight available chlorine.

2. A method according to Claim 1, wherein more than one chlorine release agent is employed, the total amount of chlorinating agent present giving from 0.5 to 100 ppm by weight available chlorine.

3. A method according to Claim 1 or Claim 2, wherein said chlorine release agent or agents is or are present in an amount to give from 3 to 20 ppm available chlorine.

4. An isotonic buffered saline contact lens-disinfecting solution containing as a chlorine release agent 4-carboxyl phenyl N-chlorosulphonamide or a salt thereof in an amount giving from 0.5 to 100 ppm by weight available chlorine.

5. A tablet or capsule for dissolution in 10 cm³ of a pharmaceutically acceptable aqueous liquid to produce a contact lens-disinfecting solution, which tablet or capsule contains an amount of 4-carboxyl phenyl N-chlorosulphonamide or a salt thereof as a chlorine release agent, sufficient to produce a concentration of the chlorine release agent giving from 0.5 to 10 ppm by weight available chlorine, when the tablet is dissolved in the said aqueous liquid.

6. A two-part pack which comprises a plurality of sachets each containing a predetermined quantity of a sterile, isotonic buffered saline and a plurality of tablets or capsules, each of said tablets or capsules being soluble in the quantity of saline contained within a said sachet and containing as a chlorine release agent 4-carboxyl phenyl N-chlorosulphonamide or a salt thereof, in an amount to produce with said quantity of saline a contact lens disinfecting solution containing from 0.5 to 100 ppm by weight available chlorine.

7. A method of producing a pharmaceutically acceptable contact lens-disinfecting solution which comprises dissolving in a predetermined quantity of a sterile isotonic buffered saline a tablet or capsule containing as a chlorine release agent 4-carboxyl phenyl N-chlorosulphonamide or a salt thereof, in an amount to produce with said quantity of liquid a contact lens treating solution having from 0.5 to 100 ppm by weight available chlorine.

## Patentansprüche

1. Verfahren zum Desinfizieren von Kontaktlinsen durch Behandeln der Linsen mit einer wäßrigen Lösung, die 4-Carboxylphenyl-N-chlorsulphonamid oder ein Salz desselben als chlorfreisetzendes Mittel in einer Menge, die von 0,5-100 ppm Gewicht erhältliches Chlor ergibt, enthält.

2. Verfahren nach Anspruch 1,
**dadurch gekennzeichnet,**
daß mehr als ein chlorfreisetzendes Mittel verwendet wird, wobei die Gesamtmenge der vorhandenen Chlorierungsmittel von 0,5-100 ppm Gewicht erhältliches Chlor ergibt.

3. Verfahren nach Anspruch 1 oder 2,
**dadurch gekennzeichnet,**
daß das(die) chlorfreisetzende(n) Mittel in einer Menge vorhanden ist(sind), die von 3-20 ppm erhältliches Chlor ergibt.

4. Isotonische gepufferte Salzlösung zum Desinfizieren von Kontaktlinsen, die 4-Carboxylphenyl-N-chlorsulphonamid oder ein Salz desselben als chlorfreisetzendes Mittel in einer Menge, die von 0,5-100 ppm Gewicht erhältliches Chlor ergibt, enthält.

5. Tablette oder Kapsel zum Auflösen in 10 cm³ einer pharmazeutisch verträglichen wäßrigen Flüssigkeit, um eine Desinfektionslösung für Kontaktlinsen herzustellen, wobei die Tablette oder Kapsel eine solche Menge von 4-Carboxylphenyl-N-chlorsulphonamid oder ein Salz desselben als chlorfreisetzendes Mittel enthält, die ausreichend ist, um beim Auflösen der Tablette in der wäßrigen Flüssigkeit eine Konzentration des chlorfreisetzenden Mittels zu erzeugen, die 0,5-10 ppm Gewicht erhältliches Chlor ergibt.

6. Zwei-Komponenten Packung mit einer Vielzahl von Packungen, die jeweils eine vorbestimmte Menge einer sterilen isotonischen gepufferten Salzlösung und eine Vielzahl von Tabletten oder Kapseln enthalten, wobei jede der Tabletten oder Kapseln in der Menge Salzlösung, die in einer solchen Packung enthalten ist, löslich ist und 4-Carboxylphenyl-N-chlorsulphonamid oder ein Salz desselben als chlorfreisetzendes Mittel in einer Menge enthält, um mit der Menge von Salzlösung eine Kontaktlinsendesinfektionslösung zu schaffen, die von 0,5-100 ppm Gewicht erhältliches Chlor aufweist.

7. Verfahren zum Herstellen einer pharmazeutisch verträglichen Desinfektionslösung für Kontaktlinsen durch Auflösen einer Tablette oder Kapsel, die 4-Carboxylphenyl-Nchlorsulphonamid oder ein Salz desselben als chlorfreisetzendes Mittel enthält, in einer vorbestimten Menge einer sterilen isotonischen gepufferten Salzlösung, wobei die Menge an 4-Carboxylphenyl-N-chlorsulphonamid in der Tablette oder Kapsel so ist, um mit der Flüssigkeitsmenge eine Behandlungslösung für Kontaktlinsen zu schaffen mit von 0,5-100 ppm Gewicht erhältlichem Chlor.

## Revendications

1. Méthode de désinfection de lentilles de contact comprenant le traitement des lentilles avec une solution aqueuse contenant comme agent libérant du chlore le 4-carboxylphényl-N-chlorosulfonamide ou un sel de ce composé, ledit agent libérant du chlore étant présent à une quantité donnant entre 0,5 et 100 ppm en poids de chlore disponible.

2. Méthode conforme à la revendication 1, dans laquelle on utilise plus d'un seul agent libérant du chlore, la quantité totale d'agents libérant du chlore présents donnant entre 0,5 et 100 ppm en poids de chlore disponible.

3. Méthode conforme à la revendication 1 ou 2, dans laquelle le(s)dit(s) agent(s) libérant du chlore est ou sont présents en une quantité donnant entre 3 et 20 ppm en poids de chlore disponible.

4. Solution saline, isotonique, tamponnée pour la désinfection de lentilles de contact, contenant comme agent libérant du chlore le 4-carboxylphényl-N-chlorosulfonamide ou un sel de ce composé, en une quantité donnant entre 0,5 et 100 ppm en poids de chlore disponible.

5. Comprimé ou capsule pour la dissolution dans 10 cm³ d'un liquide acceptable d'un point de vue pharmaceutique, afin de préparer une solution désinfectante de lentilles de contact, lesdits comprimé ou capsule contenant comme agent libérant du chlore une quantité suffisante de 4-carboxylphényl-N-chlorosulfonamide ou d'un sel de ce composé pour obtenir une concentration en agent libérant du chlore donnant entre 0,5 et 100 ppm en poids de chlore disponible, lorsqu'on dissout le comprimé dans ledit liquide aqueux.

6. Emballage en deux parties comprenant plusieurs flacons dont chacun contient une quantité prédéterminée d'une solution saline, isotonique, tamponnée, stérile et plusieurs comprimés ou capsules, dont chacun(e) est soluble dans la quantité de solution saline contenue dans lesdits flacons et contient, comme agent libérant du chlore, du 4-carboxylphényl-N-chlorosulfonamide ou un sel de ce composé, en une quantité telle qu'il donne avec ladite quantité de solution saline une solution désinfectante pour lentilles de contact contenant entre 0,5 et 100 ppm en poids de chlore disponible.

7. Méthode de préparation d'une solution de désinfection de lentilles de contact acceptable d'un point de vue pharmaceutique, comprenant la dissolution, dans une quantité prédéterminée d'une solution saline, isotonique, tamponnée, stérile, d'un comprimé ou d'une capsule contenant, comme agent libérant du chlore, le 4-carboxylphényl-N-chlorosulfonamide ou un sel de ce composé, en une quantité suffisante pour préparer avec ladite quantité de liquide une solution de traitement de lentilles de contact contenant entre 0,5 et 100 ppm en poids de chlore disponible.
